# EUROPEAN PATENT SPECIFICATION

(11) **EP 1 306 671 B1**
(45) Date of publication and mention of the grant of the patent: **02.06.2010**
(21) Application number: 01954389.1
(22) Date of filing: 31.07.2001
(51) Int. Cl.: G01N 33/48, G01N 33/53, G01N 33/569, C12Q 1/70

(54) **METHOD OF PRETREATING SAMPLE**
VERFAHREN ZUM VORBEHANDELN EINER PROBE
PROCEDE POUR PRETRAITER UN ECHANTILLON

(30) Priority: 01.08.2000 JP 2000233109
(43) Date of publication of application: 02.05.2003
(73) Proprietor: SYSMEX CORPORATION, Kobe-shi, Hyogo 651-0073 (JP)
(72) Inventor: SAIT0, Noriyuki, Kobe-shi, Hyogo 651-2241 (JP); IMOARAI, Takeshi, Kobe-shi, Hyogo 651-2241 (JP); AKI, Masako, Kobe-shi, Hyogo 651-2241 (JP); AMATSUJI, Yasuo, Kobe-shi, Hyogo 651-2241 (JP)
(74) Representative: Forstmeyer, Dietmar
(86) International application number: PCT/JP2001/006589
(87) International publication number: WO 2002/010744

(56) References cited:
- EP-A- 0 444 302
- EP-A- 0 584 767
- EP-A- 0 967 484
- JP-A- 6 324 040
- JP-A- 8 313 525
- JP-A- 9 501 494
- JP-A- 2000 206 115
- US-A- 4 703 001
- US-A- 4 748 109
- US-A- 4 810 635
- WAGNER ET AL: "Structural proteins of vesicular stomatitis viruses" J.VIROL., vol. 3, no. 4, 1969, pages 395-403,
- PROFUMO ET AL: "Core histone acetylation during lymphocyte activation" FEBS LETTERS, vol. 250, no. 2, July 1989 (1989-07), pages 297-300,
- CREIGHTON (ED): "Protein Structure: A Practical Approach" 1989, IRL PRESS , OXFORD, UK

## Description

### Technical field

The present invention relates to a method for a pretreatment of a test sample used for detection and measurement of a virus contained in a biosample particularly a microorganism and use of a reaction reagent for immunological measurement and is used for a so-called diagnostic drug for a clinical test.

### Background Art

In detection and measurement of a specific ingredient, which is contained in a biosample including, for example, blood, spinal fluid, seminal fluid, saliva, urine, stool, sputum, rhinorrhea, secreted liquid, sweat, and the like, it is frequently necessary to pretreat these samples to make detection and measurement of the specific ingredient easy. As measures for this purpose, various methods for pretreatment have been proposed so far. For example, it has been known that for measurement of a core antigen of a virus, a method for breaking pallium of the virus by a surfactant has been known to expose a core protein for measurement (JP P1996-50133 A and JP P1999-108932 A).

In addition, for measurement of a soluble lipopolysaccaride derived from a bacterium such as Chlamidia, a combination of an anionic polysaccharide with the surfactant has been disclosed (JP P1997-127110 A).

EP 0 967 484 refers to methods for detecting or assaying virus, wherein a treatment solution is used containing chaotropic ion and an acidifying agent.

A problem of the present invention is to provide, in detection and measurement of the ingredient contained in the biosample, particularly a microorganism ingredient, the method for pretreatment to enhance reactivity and reaction specificity.

For example, in order to detect immunologically influenza virus contained in sputum or rhinorrhea collected from a patient, sputum or rhinorrhea was necessarily treated with the pretreatment liquid to expose the antigen. In such the biosample, a large amount of a substance disturbing the measurement is contained and, therefore, the pretreatment is required to enhance reactivity of a target substance for the measurement without a bad influence to the measurement.

### Disclosure of the Invention

As a result of intensive studies by the present inventors, we found that the problem of the present invention can be solved by treating with a liquid for pretreatment of a test sample (or reaction liquid) for measurement of the ingredient contained in the test sample and a pretreatment liquid containing at least a nonionic or amphoteric surfactant and a reducing agent, and an organic acid or a salt thereof, resulting in completion of the present invention.

The present invention includes:
1. A method for immunologically measuring virus in a test sample, comprising:
   pretreating the test sample with a reagent containing a nonionic or amphoteric surfactant, reducing substance, and an organic acid or a salt thereof, and
   measuring the virus in the pretreated test sample by immunochemical method,
   wherein the test sample is selected from the group consisting of rhinorrhea, pus, a waste liquid by washing a nasal cavity,
   a waste liquid by wiping the nasal cavity, a waste liquid by wiping a pharynx, and sputum.
2. The method according to foregoing paragraph 1, wherein the reducing substance is a reductive compound containing sulfur.
3. The method according to foregoing paragraphs 1 to 2, wherein the reducing substance is selected from the group consisting of mercaptoethylamine, mercaptoethanol, dithiothreitol, cysteine, N-acetyl-L-cysteine, 2-(2-aminoethyl)isothiourea dihydrobromide, and tris(2-carboxyethyl)phosphin.
4. The method according to any of foregoing paragraphs 1 to 3, wherein the virus is influenza virus.
5. Use of a reagent comprising a nonionic or amphoteric surfactant, reducing substance, and an organic acid or a salt thereof, for pretreating a test sample in immunological measurement of virus in the test sample, wherein the test sample is selected from the group consisting of rhinorrhea, pus, a waste liquid by washing a nasal cavity, a waste liquid by wiping the nasal cavity, a waste liquid by wiping a pharynx, and sputum.

### Best Mode for Carrying Out the Invention

In the invention, a virus antigen is measured immunologically. A specific example of the test sample includes, for example, viruses such as herpes virus (HSV, CMV, ZVZ, EBV, HHV, and the like), influenza virus, human immunodeficiency virus (HIV), human adult T - cell leukemia virus (HTLV), hepatitis virus (HBV, HCV, HDV, and HGV), and also lesion viruses of such as a cold syndrome, a digestive system disease, a central nerve system disease, a respiratory system disease, hemorrhagic fever, and other various diseases. Especially, it is preferable for measurement of an influenza antigen. Not restricted to this, it can be used for measurement of the virus antigen necessary for a pretreatment to expose the antigen.

The test sample in the invention is the ingredient contained in a biosample or the sample derived from the biosample. The biosample or the sample derived from the biosample includes pus, sputum, rhinorrhea, a waste liquid by washing a nasal cavity, the waste liquid by wiping the nasal cavity, the waste liquid by wiping a pharynx. When the specific ingredient contained in the biosample is immunologically detected and measured, the test sample is previously treated with the pretreatment solution to subject to detecting and measuring reactions.

The organic acid or the salt thereof used in the invention is not specially restricted, and, for example, one member or a combination of two or more members, which are selected from acetic acid, succinic acid, tartaric acid, citric acid and a salt thereof, is used. The organic acid or the salt thereof, which is used in the invention, may be used singly or by blending two or more members of them. As other organic acids, oxalic acid, glycolic acid, gluconic acid, malic acid, and the like are exemplified.

The surfactant used is a nonionic surfactant or an amphoteric surfactant in the invention. The surfactant used is not specially restricted, and representative examples include polyoxyethylene alkyl phenyl ether, polyoxyethylene alkyl ether, polyoxyethylene sorbitan alkyl ester, alkyl pyridinium salt, higher alcohol sulfate ester salt, and the like.

The reductive substance used in the invention is a reducing compound containing sulfur and used singly or in blend of two or more members. A representative reductant includes reductive compounds containing sulfur, such as mercaptoethylamine, mercaptoethanol, dithiothreitol, cysteine, N-acetyl-L-cysteine, 2-(2-aminoethyl)isothiourea dihydrobromide, tris(2-carboxyethyl)phosphin, a hydrosulfite salt, a sulfite, and the like.

An amount for use of these substances in pretreatment is determined as a concentration in a sulution of the test sample.

The added amount of the organic acids in total is a minimal 5 mM or higher, preferably ranges 10 to 500 mM, and more preferably ranges from 50 to 100 mM. Adding 100 mM or higher amount yields no special effect of the treatment, but the amount may be used. The added amount of the surfactant in total is 0.01 w/v% or larger, preferably 0.05 w/v% or larger, and an upper limit is 5 w/v%, preferably 1 w/v%, and more preferably 0.125 w/v%. Adding 0.125 w/v% or higher amount yields no special effect of the treatment, but the amount may be used. The added amount of the reductants in total is a minimal 0.5 mM or higher, preferably ranges 1 to 500 mM, and more preferably ranges from 5 to 50 mM. Adding 10 mM or higher amount yields no special effect of the treatment, but the amount may be used.

In an example of specific embodiment according to the invention, such a solution is used as the pretreatment solution that contains 0.01 to 5 w/v%, more preferably 0.05 to 1.0 w/v% of polyoxyethylene nonylphenyl ether (commercial name NP-40), which is the nonionic surfactant, is used as the surfactant, 1 to 100 mM, more preferably 10 to 50 mM of a hydrochloric acid salt of 2-mercaptoethylamine is used as the reductant, 10 to 500 mM, more preferably 50 to 100 mM of citric acid is used as the organic acid, and that has pH adjusted to 5 to 7, more preferably about 6.

The test sample in the invention is measured by immunochemical method following the pretreatment, for example, through blending 100µL of the pretreatment solution with a 20 µL of a patient's rhinorrhea containing influenza virus. The method is particularly exemplified by sandwich enzyme immunossay method by using an anti-influenza monoclonal antibody or particle-labeling immunochromatographic method through labeling the anti-influenza monoclonal antibody with a colored latex particle, and the like.

### Example

The invention will be described with examples below and the present invention is not restricted to these examples.

### Example 1

Various surfactants were added to a 20 mM phosphate buffer solution (pH 6.0) to prepare pretreatment solutions. Following blending 100 µL of each of the pretreatment solution with cultured influenza virus to treat at an ordinary temperature for 10 min, a virus antigen was measured by the enzyme immunossay method by using the anti-influenza virus monoclonal antibody. The result will be presented in Table 1

**Table 1**

| Effect of pretreatment for influenza virus measurement using various surfactants | | | | | |
|---|---|---|---|---|---|
| | | | (Absorbency at 492 nm) | | |
| Surfactant | | | Concentration (W/V%) | | |
| | 0 | 0.06 | 0.125 | 0.25 | 0.5 |
| Nonidet P-40 | 0.101 | 0.788 | 0.762 | 0.758 | 0.839 |
| Triton X-100 | 0.101 | 0.710 | 0.748 | 0.725 | 0.733 |
| Tween 80 | 0.101 | 0.169 | 0.196 | 0.216 | 0.267 |
| Tween 20 | 0.101 | 0.623 | 0.606 | 0.562 | 0.580 |
| Nonion HS-210 | 0.101 | 0.778 | 0.783 | 0.749 | 0.644 |
| Nonion HS-240 | 0.101 | 0.129 | 0.132 | 0.125 | 0.131 |
| Nonion A10-R | 0.101 | 0.849 | 0.819 | 0.816 | 0.875 |
| Emergen 909 | 0.101 | 0.753 | 0.757 | 0.771 | 0.754 |
| Bridge 35 | 0.101 | 0.531 | 0.533 | 0.527 | 0.573 |
| Bridge 58 | 0.101 | 0.095 | 0.322 | 0.415 | 0.396 |
| Bridge 76 | 0.101 | 0.675 | 0.686 | 0.652 | 0.725 |
| Bridge 97 | 0.101 | 0.713 | 0.730 | 0.687 | 0.729 |
| Bridge 98 | 0.101 | 0.588 | 0.341 | 0.598 | 0.530 |
| Bridge 721 | 0.101 | 0.135 | 0.313 | 0.403 | 0.391 |
| CHAPS | 0.101 | 0.125 | 0.161 | 0.383 | 0.552 |
| CHAPSO | 0.101 | 0.132 | 0.209 | 0.477 | 0.541 |
| Octylglucoside | 0.101 | 0.115 | 0.111 | 0.117 | 0.585 |
| Octylthioglucoside | 0.101 | 0.128 | 0.142 | 0.615 | 0.840 |

As the result of the above experiment, all examined surfactants showed that intensity of a measurement signal in the enzyme immunossay method enhances in a concentration range at least from 0.06 to 0.5 w/v%, which expresses clearly the effect of the pretreatment.

### Example 2

Except for addition of various reductants to the 20 mM phosphate buffer sulution containing 0.1 w/v% Nonidet P-40, the operation was conducted in the same way as that in Example 1 to test the effect of reductants. The result will be presented in Table 2.

**Table 2**

| Effect of pretreatment by various reductants | | | | |
|---|---|---|---|---|
| (Absorbency at 492 nm) | | | | |
| Reductant | Concetration (mM) | | | |
| | 0 | 2 | 10 | 50 |
| N-acetyl-L-cysteine | 0.129 | 0.445 | 0.916 | 0.998 |
| 2-(2-aminoethyl)isothiourea dihydrobromide | 0.129 | 1.021 | 1.209 | 1.411 |
| 2-mercaptoethylamine hydrochloride | 0.129 | 1.201 | 1.505 | 1.554 |
| tris(2-carboxyethyl)phosphin | 0.129 | 0.372 | 0.674 | 0.902 |
| Dithiothreitol | 0.129 | 0.860 | 1.010 | 0.908 |

As the result of the above experiment, it was found that by adding reductants, a larger absorbency was observed and a large effect of the pretreatment was yielded.

### Example 3

Except for addition of various organic acids to the 20 mM phosphate buffer solution containing 0.1 w/v% Nonidet P-40 and 20 mM 2-mercaptoethylamine hydrochloride, the operation was conducted in the same way as that in Example 1 to test the effect of organic acids. The result will be presented in Table 3.

**Table 3**

| The effect of organic acids | | | | | |
|---|---|---|---|---|---|
| | (Absorbency at 492 nm) | | | | |
| | Concentration (mM) | | | | |
| | 0 | 10 | 50 | 100 | 200 |
| Citric acid | 0.125 | 0.325 | 0.415 | 0.422 | 0.425 |
| Succinic acid | 0.125 | 0.154 | 0.189 | 0.203 | 0.204 |
| Acetic acid | 0.125 | 0.204 | 0.216 | 0.243 | 0.245 |
| Oxalic acid | 0.125 | 0.168 | 0.199 | 0.211 | 0.209 |

As the result of the above experiment, it was found that by adding organic acids, a larger signal was observed and the effect of the pretreatment was high.

### Example 4

The following pretreatment solution was prepared: the 20 mM phosphate buffer solution (pH 6.0, 0.1% ONP /NaPB) containing 0.1 w/v% Nonidet P-40; a solution (NP40+NAC/NaPB) prepared by adding 10 mM N-acetyl-L-cysteine to 20 mM phosphate buffer solution (pH 6.0) containing 0.1 w/v% Nonidet P-40; a solution (NP40+NAC/ citrate) prepared by adding 10 mM N-acetyl-L-cysteine to 100 mM citric acid buffer solution (pH 6.0) containing 0.1 w/v% Nonidet P-40; and a solution (NP40+MEA/citrate) prepared by adding 10 mM 2-mercaptoethylamine hydrochloride to 100 mM citric acid buffer solution (pH 6.0) containing 0.1 w/v% Nonidet P-40. Then, each sample of rhinorrhea or the waste liquid by wiping the pharynx (No. 5x2, No. 10, No. 19, No. 20, and No. 31) of the influenza patient, the cultured virus antigen (NIBSC Corp. made), and a commercial influenza antigen (A/TexasI/77 Chemicon Corp. made) was pretreated and then, an influenza antigen was measured by the immunoassay method using the anti-influenza virus monoclonal antibody. The result will be presented in Table 4.

**Table 4**

| The result of measurement of the influenza antigen using various pretreatment solutions | | | | | | | |
|---|---|---|---|---|---|---|---|
| | (Absorbency at 492 nm) | | | | | | |
| Pretreatment solution | No5 x 2 | No. 10 | No. 19 | No. 20 | No. 31 | Sydney | CHEMICON |
| 0.1%NP40/NaPB | 0.29 | 0.038 | 0.002 | 0.224 | 0.135 | 0.485 | 0.089 |
| NP40+NAC/NaPB | 0.330 | 0.079 | 0.034 | 0.254 | 0.057 | 0.554 | 0.418 |
| NP40+NAC/Citrate | 0.386 | 0.094 | 0.036 | 0.423 | 0.25 | 0.798 | 0.808 |
| NP40+AET/Citrate | 0.448 | 0.09 | 0.053 | 0.466 | 0.193 | 0.78 | 0.861 |
| NP40+MEA/Citrate | 0.434 | 0.095 | 0.064 | 0.435 | 0.235 | 0.779 | 1.224 |

From the result as described above, it was found that in comparison with the sample treated with the pretreatment solution of the 20 mM phosphate buffer solution (pH 6.0, 0.1% NP/NaPB), which contains 0.1 w/v% Nonidet P-40, and the pretreatment solution, to which the reductant NAC was added, the sample treated with the pretreatment solution, to which the organic acid was added, yielded the larger signal and the effect of the pretreatment was high.

### Effect of the Invention

Through a treatment with the liquid for pretreatment of the test sample (or reaction liquid) for measurement of the component contained in the test sample and the pretreatment liquid containing the surfactant and the reducing agent, and the organic acid or the salt thereof, reactivity and reaction specificity are enhanced in detection and measurement of an ingredient, particularly a virus, contained in a biosample

## Claims

1. A method for immunologically measuring virus in a test sample, comprising:
pretreating the test sample with a reagent containing a nonionic or amphoteric surfactant, reducing substance, and an organic acid or a salt thereof, and
measuring the virus in the pretreated test sample by immunochemical method,
wherein the test sample is selected from the group consisting of rhinorrhea, pus, a waste liquid by washing a nasal cavity,
a waste liquid by wiping the nasal cavity, a waste liquid by wiping a pharynx, and sputum.

2. The method according to claim 1, wherein the reducing substance is a reductive compound containing sulfur.

3. The method according to claim 1 to 2, wherein the reducing substance is selected from the group consisting of mercaptoethylamine, mercaptoethanol, dithiothreitol, cysteine, N-acetyl-L-cysteine, 2-(2-aminoethyl)isothiourea dihydrobromide, and tris(2-carboxyethyl)phosphin.

4. The method according to any of claims 1 to 3, wherein the virus is influenza virus.

5. Use of a reagent comprising a nonionic or amphoteric surfactant, reducing substance, and an organic acid or a salt thereof, for pretreating a test sample in immunological measurement of virus in the test sample, wherein the test sample is selected from the group consisting of rhinorrhea, pus, a waste liquid by washing a nasal cavity, a waste liquid by wiping the nasal cavity, a waste liquid by wiping a pharynx, and sputum.

## Patentansprüche

1. Verfahren zur immunologischen Messung eines Virus in einer Testprobe, umfassend:
die Vorbehandlung der Testprobe mit einem Reagenz, das ein nicht-ionisches oder amphoteres grenzflächenaktives Mittel, eine reduzierende Substanz, und eine organische Säure oder ein Salz derselben enthält, und
das Messen des Virus in der vorbehandelten Testprobe durch ein immunchemisches Verfahren,
wobei die Testprobe aus der Gruppe ausgewählt wird, welche aus Fließschnupfen, Eiter, einer Flüssigkeit, die durch Waschen der Nasenhöhle erhalten wird, einer Flüssigkeit, die durch Wischen der Nasenhöhle erhalten wird, einer Flüssigkeit, die durch Wischen des Rachens erhalten wird, und Speichel besteht.

2. Verfahren nach Anspruch 1, wobei die reduzierende Substanz eine reduzierende Verbindung darstellt, die Schwefel enthält.

3. Verfahren nach Anspruch 1 oder 2, wobei die reduzierende Substanz aus der Gruppe ausgewählt wird, welche aus Mercaptoethylamin, Mercaptoethanol, Dithiothreit, Cystein, N-Acetyl-L-cystein, 2-(2-Aminoethyl)isothioharnstoff-dihydrobromid und Tris-(2-carboxyethyl)phosphin besteht.

4. Verfahren nach einem der Ansprüche 1 bis 3, wobei das Virus ein Influenza-Virus ist.

5. Verwendung eines Reagenz, das ein nicht-ionisches oder amphoteres grenzflächenaktives Mittel, eine reduzierende Substanz, und eine organische Säure oder ein Salz derselben umfasst, zur Vorbehandlung einer Testprobe in einer immunologischen Messung eines Virus in der Testprobe, wobei die Testprobe aus der Gruppe ausgewählt wird, welche aus Fließschnupfen, Eiter, einer Flüssigkeit, die durch Waschen der Nasenhöhle erhalten wird, einer Flüssigkeit, die durch Wischen der Nasenhöhle erhalten wird, einer Flüssigkeit, die durch Wischen des Rachens erhalten wird, und Speichel besteht.

## Revendications

1. Procédé de mesure immunologique d'un virus dans un échantillon à analyser, qui comprend :
le prétraitement de l'échantillon à analyser avec un réactif qui contient un surfactant non ionique ou amphotère, une substance réductrice et un acide organique ou un sel de celui-ci, et
la mesure du virus dans l'échantillon à analyser prétraité par un procédé immunochimique,
l'échantillon à analyser étant choisi dans le groupe constitué par une rhinorrhée, du pus, un liquide résiduel obtenu par lavage de la cavité nasale, un liquide résiduel obtenu par essuyage de la cavité nasale, un liquide résiduel obtenu par essuyage du pharynx et des expectorations.

2. Procédé selon la revendication 1, dans lequel la substance réductrice est un composé réducteur contenant du soufre.

3. Procédé selon la revendication 1 ou 2, dans lequel la substance réductrice est choisie dans le groupe constitué par la mercaptoéthylamine, le mercaptoéthanol, le dithiothréitol, la cystéine, la N-acétyl-L-cystéine, la dihydrobromure d'amino-2-éthylisothiourée et la Tris(2-carboxyéthyl)phosphine.

4. Procédé selon l'une quelconque des revendications 1 à 3, le virus étant le virus de la grippe.

5. Utilisation d'un réactif qui comprend un surfactant non ionique ou amphotère, une substance réductrice et un acide organique ou un sel de celui-ci, pour prétraiter un échantillon à analyser par mesure immunologique d'un virus dans l'échantillon à analyser, l'échantillon à analyser étant choisi dans le groupe constitué par une rhinorrhée, du pus, un liquide résiduel obtenu par lavage de la cavité nasale, un liquide résiduel obtenu par essuyage de la cavité nasale, un liquide résiduel obtenu par essuyage du pharynx et des expectorations.
